# NEUE EUROPÄISCHE PATENTSCHRIFT

(11) **EP 1 098 602 B2**
(45) Veröffentlichungstag und Bekanntmachung des Hinweises auf die Entscheidung über den Einspruch: **25.08.2010**
(45) Hinweis auf die Patenterteilung: 25.06.2003
(21) Anmeldenummer: 99938239.3
(22) Anmeldetag: 14.07.1999
(51) Int. Cl.: A61B 17/80, F16B 43/02

(54) **BEFESTIGUNGSANORDNUNG**
FIXING DEVICE
DISPOSITIF DE FIXATION

(30) Priorität: 20.07.1998 DE 19832513
(43) Veröffentlichungstag der Anmeldung: 16.05.2001
(73) Patentinhaber: BIEDERMANN MOTECH GmbH, 78054 Villingen-Schwenningen (DE)
(72) Erfinder: EBERLEIN, Roland, D-09399 Niederwürschnitz (DE); WILBERG, Lothar, D-74424 Bühlertann (DE)
(74) Vertreter: Hofer, Dorothea
(86) Internationale Anmeldenummer: PCT/EP1999/004973
(87) Internationale Veröffentlichungsnummer: WO 2000/004836

(56) Entgegenhaltungen:
- EP-A- 0 809 975
- EP-B1- 0 355 035
- DE-C- 3 420 696
- GB-A- 2 305 483
- US-A- 4 388 921
- US-A- 5 057 111
- US-A- 5 735 853

## Beschreibung

Die Erfindung betrifft eine Befestigungsanordnung zum Befestigen eines Befestigungsteils, beispielsweise einer Platte, eines Riegels, eines Winkels, eines Profils, eines Beschlags, eines Halters oder dergleichen, an einem Untergrund mittels zumindest einer Schraube, die durch zumindest eine Bohrung in dem Befestigungsteil durchführbar und in den Untergrund einschraubbar ist, nach dem Oberbegriff des Anspruchs 1.

Eine derartige Befestigungsanordnung mit einer unmittelbar in der Bohrung gelagerten verkippbaren Hülse ist aus der US-A-4 388 921 bekannt.

Befestigungsanordnungen der eingangs genannten Art können für die unterschiedlichsten Anwendungen vorgesehen sein.

Beispielsweise werden solche Befestigungsanordnungen in häuslichen Anwendungen dazu verwendet, um beispielsweise Gegenstände, wie Lampen, Regale oder dergleichen, an einer Wand oder einer Decke anzubringen. Für solche Gegenstände sind meistens Befestigungsteile in Form von Wandbeschlägen, Profilen oder dergleichen vorgesehen, die an der Wand oder der Decke mittels einer oder mehrerer Schrauben befestigt werden müssen. Derartige Befestigungsteile weisen in der Regel eine oder mehrere Bohrungen auf, durch die eine Schraube durchgeführt und in den Untergrund eingeschraubt wird. Dazu wird bei diesen Anwendungsfällen zunächst in die Wand bzw. die Decke ein Loch gebohrt, in das ein Dübel eingesetzt wird, in den dann die Schraube eingedreht wird. Durch vollständiges Eindrehen und Festziehen der Schraube drückt dann der Schraubenkopf das Befestigungsteil gegen den Untergrund.

Bei häuslichen Anwendungen tritt aufgrund mangelnder Übung des Anwenders oder aufgrund unzulänglicher Werkzeuge häufig der Fall ein, daß das Loch in der Wand oder der Decke nicht lotrecht zur Oberfläche, sondern schräg gebohrt wird. Insbesondere nahe bei Raumecken ist es besonders schwierig, ein Loch lotrecht in die Wand oder Decke zu bohren, weil das Gehäuse der Bohrmaschine ein lotrechtes Ansetzen des Bohrers an der Decke bzw. der Wand behindert oder unmöglich macht.

Wenn das Loch in der Wand oder der Decke schräg gebohrt wurde, muß die Schraube dann zwangsläufig schräg durch die Bohrung in dem Befestigungsteil in den Untergrund eingedreht werden. Da der Schraubenschaft der verwendeten Schraube in der Regel einen Durchmesser aufweist, der nur geringfügig kleiner ist als die Bohrung, kann sich der Schaft der Schraube beim Eindrehen mit dem oberen und/oder unteren Rand der Bohrung verkeilen, so daß es nur mit erhöhtem Kraftaufwand möglich ist, die Schraube vollständig einzudrehen. Im schlimmsten Fall kann es sogar unmöglich werden, die Schraube vollständig einzudrehen, wodurch das Befestigungsteil dann nicht fest und unbeweglich an dem Untergrund befestigt werden kann. Auch durch eine längliche Ausbildung der Bohrung kann dieser Nachteil nicht vermieden werden.

Selbst wenn es gelingt, die Schraube dennoch vollständig in den Untergrund einzudrehen und festzuziehen, bildet der Schraubenkopf mit dem Rand der Bohrung oder im Falle von Senkkopfschrauben in der Bohrung unter Umständen nur eine punktförmige Anlage mit dem Befestigungsteil, die ein spielfreies Befestigen des Befestigungsteiles an dem Untergrund nicht gewährleistet.

Ein weiterer Anwendungsfall derartiger Befestigungsanordnungen besteht bei der Selbstmontage von Möbeln, beispielsweise beim Anbringen von Scharnieren für Schranktüren oder dergleichen. Bei diesen Anwendungsfällen werden als Schrauben selbstschneidende Holzschrauben verwendet. Beim Anbringen eines Winkels ist es jedoch ebenfalls häufig schwierig, die Holzschraube aufgrund des erschwerten Zugangs im Bereich des Winkels lotrecht zur Schrankwand einzuschrauben. Sobald die Schraube wenige Gewindegänge in den Untergrund eingeschraubt ist und somit in dem Untergrund greift, ist die Orientierung des Schafts bezüglich dem Untergrund und damit bezüglich dem Befestigungsteil meist unkorrigierbar vorgegeben. Auch hier tritt dann das gleiche Problem auf, daß sich die Schrauben nicht vollständig oder nur mit erhöhtem Kraftaufwand festdrehen lassen, um das Befestigungsteil unbeweglich mit dem Untergrund zu verbinden.

Ein weiterer bedeutsamer Anwendungsfall der eingangs genannten Befestigungsanordnung liegt im medizinischen Bereich. Zur Heilung von Knochenfrakturen werden dem Patienten zur Fixierung der gebrochenen Knochenteile Riegel implantiert, die aus einer länglichen metallischen Platte gebildet werden. In dem Riegel sind zumindest zwei Bohrungen vorgesehen, so daß der Riegel die Fraktur überspannend mit beiden Knochenteilen verschraubt werden kann. Als Schrauben werden Knochenschrauben verwendet, die unmittelbar in den Knochen eingedreht werden. Aufgrund anatomischer Gegebenheiten des Patienten bzw. der Stelle der Fraktur des Knochens ist es hier oft sogar erforderlich, die Knochenschrauben schräg zu dem Knochenriegel in den Knochen einzudrehen, woraus sich auch hier die im Zusammenhang mit den technischen Anwendungen erörterten Probleme ergeben.

Hinzu kommt bei diesem Anwendungsfall, daß die Knochenteile, an denen der Riegel befestigt werden muß, eine nichtplane, ungleichmäßige Oberfläche mit konkaven und konvexen Wölbungen aufweisen. Um den Knochenriegel unbeweglich an den Knochenteilen zu fixieren, dürfen die Knochenschrauben jedoch nicht mit Gewalt in den Knochen eingeschraubt werden, da dadurch unerwünschte Verspannungen oder sogar ein zusätzlicher Bruch der Knochenteile auftreten können. Es ist daher erforderlich, daß die Knochenschrauben eine unbewegliche Verbindung des Knochenriegels mit den Knochenteilen herstellen, ohne daß dazu eine übermäßige Kraft ausgeübt werden muß, durch die ansonsten die Knochenteile gegen den Knochenriegel gezogen und dadurch verspannt würden.

Die aus der US-A-4 388 921 bekannte Befestigungsanordnung, die für medizinische Anwendungen vorgesehen ist, beseitigt die vorstehend genannten Probleme. Diese bekannte Befestigungsanordnung (Fig. 13 in diesem Dokument) weist einen Knochenriegel mit zumindest einer Bohrung darin, eine Knochenschraube sowie eine in der Bohrung in mehreren Raumrichtungen verkippbar gelagerte Hülse auf. Die Innenfläche der Bohrung und die Außenfläche der Hülse sind sphärisch gewölbt. Allerdings befindet sich der größte Durchmesser der Bohrung an deren oberem Rand. Die Hülse ist somit in der Bohrung lose und kann herausfallen, solange die Schraube noch nicht durch die Hülse hindurchgeführt und in den Untergrund eingeschraubt ist. Die Handhabung dieser bekannten Befestigungsanordnung ist dadurch erschwert, weil der Anwender stets mit drei Teilen, nämlich dem Knochenriegel, der Knochenschraube und der Hülse hantieren und darauf achtgeben muß, daß die Hülse nicht aus der Bohrung herausfällt.

Damit vergleichbare Befestigungsanordnungen sind aus der GB-A-2 305 483 und der DE-C-34 20 696 bekannt.

Aus der EP-A-0 809 975 ist eine Befestigungsanordnung bekannt, die einen Dübel aufweist, der durch eine Bohrung in einem Knochenriegel geführt und in einen Knochen eingeschraubt werden kann, wozu der Dübel auf seiner Außenseite ein Gewinde aufweist. Der nach dem Einschrauben des Dübels in der Bohrung zu liegen kommende Kopf des Dübels ist als Aufnahme für eine Schraube ausgebildet, so daß beim Eindrehen der Schraube der Dübel gespreizt und dadurch im Knochen und in dem Knochenriegel verankert werden kann. Der Dübel selbst ist radial mit geringfügiger Kraft komprimierbar und somit ebenfalls nicht unverlierbar in der Bohrung gelagert, sofern die zuvor genannte Schraube noch nicht in den Dübel eingeschraubt ist.

Aus der US-A-5 057 111 ist eine Befestigungsanordnung bekannt, die eine in dem Befestigungsteil ebenfalls in mehreren Raumrichtungen verkippbar gelagerte Hülse aufweist, die jedoch wie bei den zuvor genannten bekannten Befestigungsanordnungen herausfallen kann.

Schließlich ist aus der EP 0 355 035 B1 ist eine Befestigungsvorrichtung nach dem Oberbegriff des Patentanspruchs 1 bekannt. Bei der Befestigungsvorrichtung gemäß der EP 0 355 035 B1 wird ein geschlitztes Einsatzteil durch den konischen Schraubenkopf aufgespreizt und in der Bohrung verklemmt. Bei einer solchen Konstruktion ist es schwierig, die Platte eng an den Knochen anzuziehen, weil es schon vorher zu einer Verklemmung kommen kann.

All die zuvor genannten bekannten Befestigungsanordnungen sind somit hinsichtlich ihrer Handhabung in der ein oder anderen Weise nachteilig.

Der Erfindung liegt daher die Aufgabe zugrunde, eine Befestigungsanordnung der eingangs genannten Art dahingehend weiterzubilden, daß ihre Handhabungseingenschaften verbessert sind.

Die Aufgabe wird gelöst durch eine Befestigungsanordnung nach Patentanspruch 1.

Bei der erfindungsgemäßen Befestigungsanordnung ist in der zumindest einen Bohrung des Befestigungsteils eine Hülse aufgenommen, die in mehreren Raumrichtungen verkippbar in der Bohrung gelagert ist. Durch diese in der Bohrung mit anderen Worten taumelnd angeordnete Hülse wird die Schraube zum Eindrehen in den Untergrund durchgeführt. Aufgrund des Vorhandenseins der taumelnd gelagerten Hülse kann die Schraube in mehreren Richtungen schräg durch das Befestigungsteil durchgeführt werden, ohne daß sich die Schraube beim Durchtritt durch die Hülse mit dieser verklemmen kann, weil die Hülse sich dann ebenfalls entsprechend schräg stellt, so daß die Längsachse der Schraube stets im wesentlichen senkrecht zur oberen und unteren Öffnung der Hülse verläuft. Dadurch wird ein Verkanten des Schafts der Schraube mit dem oberen oder dem unteren Rand der Hülse vermieden. Weiterhin ist vorgesehen, daß die Hülse eine Aufnahme zum zumindest teilweisen Aufnehmen eines Kopfes der Schraube aufweist. Dadurch wird erreicht, daß beim Festziehen der Schraube der Kopf zumindest teilweise mit der Hülse in Eingriff kommt, beispielsweise mit dieser zumindest teilweise flächig in Anlage kommt. Sobald der Kopf der Schraube mit der Aufnahme der Hülse zumindest teilweise in Anlage oder Eingriff kommt, wird die Hülse in der Bohrung fixiert und bilden der Kopf der Schraube und die Hülse eine starre unbewegliche Verbindung des Befestigungsteiles mit dem Untergrund. Es ist dabei nicht erforderlich, die Schraube zur Erreichung der unbeweglichen starren Verbindung des Befestigungsteiles mit dem Untergrund mit Gewalt anzuziehen, weil durch den Eingriff des Kopfes mit der Hülse unabhängig von der Orientierung der Schraube bezüglich der Bohrung in dem Befestigungsteil eine sichere Verankerung der Schraube in dem Befestigungsteil erreicht wird.

Bei der erfindungsgemäßen Befestigungsanordnung sind eine Innenfläche der Bohrung und eine Außerfläche der Hülse etwa sphärisch gewölbt ausgebildet, wobei sich erfindungsgemäß ein Bereich größten Durchmessers der Innenfläche und der Außenfläche zwischen einem oberen Rand der Bohrung und einem unteren Rand der Bohrung befindet, wobei die Hülse in die Bohrung eingepreßt ist. Durch diese Ausgestaltung von Hülse einerseits und Bohrung andererseits wird eine unverlierbare Aufnahme der Hülse in der Bohrung erzielt. Dadurch wird das Befestigen des Befestigungsteiles auf dem Untergrund erleichtert, da die Hülse beim Anbringen des Befestigungsteiles auf dem Untergrund nicht von Hand in der Bohrung gehalten werden muß.

In einer bevorzugten Ausgestaltung ist eine Symmetrieachse der Hülse gegen eine Symmetrieachse der Bohrung über vorzugsweise einen vollen Azimutalwinkel von 360° um einen Winkel in einem Winkelbereich von 0° bis zumindest 45° verkippbar.

Wenn die Hülse in dem zuvor genannten Raumwinkelbereich verkippbar in der Bohrung angeordnet ist, kann mit der erfindungsgemäßen Befestigungsanordnung bei allen auftretenden Fällen, in denen die Schraube beabsichtigt oder unbeabsichtigt schräg zur Bohrung orientiert ist, diese in den Untergrund problemlos eingedreht werden.

Wenn die Hülse unmittelbar in der Bohrung gelagert ist, ist von Vorteil, daß die Befestigungsanordnung konstruktiv sehr einfach aufgebaut werden kann, weil keine weiteren Teile zur Lagerung der Hülse in der Bohrung vorgesehen werden müssen. Die Bohrung bildet in diesem Ausführungsbeispiel somit selbst eine Lagerfläche für die Hülse.

Gemäß der Erfindung ist die Hülse in der Bohrung formschlüssig aufgenommen.

Diese Maßnahme hat den Vorteil, daß die Hülse aufgrund der formschlüssigen Aufnahme in der Bohrung in dieser im wesentlichen spielfrei gelagert ist, wobei es weiterhin ermöglicht wird, daß die Hülse durch Formschluß in der Bohrung unverlierbar gehalten werden kann.

In einer weiteren bevorzugten Ausgestaltung bilden der Kopf der Schraube und die Aufnahme der Hülse eine im wesentlichen formschlüssige Verbindung.

Durch eine formschlüssige Ausgestaltung der Verbindung des Kopfes der Schraube mit der Aufnahme der Hülse wird beim Eindrehen der Schraube in den Untergrund mit konstruktiv einfachen Mitteln eine im wesentlichen spielfreie Verankerung des Schraubenkopfes in der Hülse und damit der Schraube insgesamt mit dem Befestigungsteil erreicht.

In einer weiteren bevorzugten Ausgestaltung sind der Kopf der Schraube und die Aufnahme der Hülse komplementär zueinander konisch ausgebildet.

Diese Maßnahme hat den Vorteil, daß die sich radial aufweitenden Abschnitte als Anlaufschräge wirken, durch die beim Eindrehen der Schraube in den Untergrund die Hülse automatisch durch das Eintauchen des Kopfes der Schraube in die Aufnahme in die paßgenaue Lage verkippt wird. Ein weiterer Vorteil besteht darin, daß beim Festziehen der Schraube Kopf und Hülse einen Preßverband bilden, durch den die Verankerung der Schraube in dem Befestigungsteil beim Festziehen weiter verbessert wird.

In einer weiteren bevorzugten Ausgestaltung ist ein oberer Rand der Aufnahme der Hülse abgerundet.

Auch durch diese Maßnahme wird an der Hülse eine Aniaufschräge ausgebildet, die ein selbständiges lagerichtiges Positionieren der Hülse bezüglich des Kopfes der Schraube ermöglicht, sobald der Kopf mit der Hülse in Eingriff kommt, und ein Verklemmen oder Blockieren der Schraube beim Eindrehen in den Untergrund verhindert.

Gemäß der Erfindung ist ein Umfangsbereich des Kopfes der Schraube als Abstützfläche ausgebildet, die sich im angezogenen Zustand der Schraube auf einer etwa komplementär zur Abstützfläche des Kopfes ausgebildeten Abstützfläche der Hülse abstützt.

Durch diese Maßnahme wird auf vorteilhafte Weise die Verankerung des Schraubenkopfes mit der Hülse verbessert.

Weiterhin ist es bevorzugt, wenn die Hülse in etwa die gleiche Höhe aufweist wie die Bohrung, und der Kopf der Schraube in etwa die gleiche Höhe aufweist wie die Aufnahme der Hülse.

Diese Maßnahme hat den Vorteil, daß die Hülse aus der Bohrung nicht hervorsteht, und daß auch der Kopf der Schraube in der Aufnahme der Hülse vollkommen versenkt werden kann, so daß der Kopf der Schraube, die Hülse und das Befestigungsteil im Bereich der Bohrung einen im wesentlichen kantenfreien, gleichmäßigen Abschluß bilden.

In einer weiteren bevorzugten Ausgestaltung ist die Hülse einstückig aus Metall oder aus Kunststoff gefertigt, oder weist einen Metallkorpus auf, wobei die Aufnahme der Hülse dann zumindest teilweise mit Kunststoff ausgekleidet ist.

Eine Ausgestaltung der Hülse aus Kunststoff hat den Vorteil, daß die Hülse mit hoher Ausprägung radial elastisch dehnbar ausgebildet werden kann, so daß durch Versenken des Kopfes der Schraube in der Aufnahme der Hülse beim Anziehen der Schraube der Kopf der Schraube in ausgeprägten kraftschlüssigen Preßsitz mit der Aufnahme der Hülse kommt und die Hülse dabei an die Bohrung ebenfalls fest anpreßt, wodurch eine vollkommen unbewegliche Fixierung des Befestigungsteiles an dem Untergrund ermöglicht wird. Aber auch bei einer aus Metall gefertigten Hülse und einer konischen Ausbildung des Kopfes der Schraube und der Aufnahme der Hülse wird ein solcher vollkommen unbeweglicher, spielfreier Preßsitz erreicht, wenn die Hülse und die Bohrung paßgenau zueinander ausgebildet sind.

In einem ersten bevorzugten Anwendungsfall wird die erfindungsgemäße Befestigungsanordnung zur Fixierung eines Knochenbruches im menschlichen Körper verwendet, wobei das Befestigungsteil eine starre Platte oder ein starrer Riegel und die zumindest eine Schraube eine Knochenschraube ist.

Bei diesem Anwendungsfall im medizinischen Bereich erweist sich die erfindungsgemäße Befestigungsanordnung als besonders vorteilhaft, da mit dieser auch ohne ein Anziehen der Knochenschrauben mit übermäßiger Kraft eine stabile, spielfreie Verankerung eines Knochenriegels an den Knochenteilen beidseits der Fraktur erreicht wird.

In weiteren bevorzugten Anwendungsfällen wird die erfindungsgemäße Befestigungsanordnung zum Anbringen eines Gegenstandes an einer Wand, wobei das Befestigungsteil ein Wandbeschlag, ein Profil oder dergleichen ist, oder zur Montage von Möbeln oder dergleichen verwendet, wobei das Befestigungsteil ein Beschlag, ein Scharnier, ein Winkel oder dergleichen ist.

Auch bei diesen Anwendungsfällen wird durch die erfindungsgemäße Befestigungsanordnung eine unbewegliche Befestigung eines Befestigungsteiles an einem Untergrund, wie einer Wand oder einer Decke, auch dann ermöglicht, wenn die Schraube bzw. die Schrauben schräg in den Untergrund eingeschraubt werden.

Ausführungsbeispiele der Erfindung sind in der Zeichnung dargestellt und werden hiernach mit Bezug auf die Figuren näher beschrieben. Es zeigen:
- Fig. 1: eine erfindungsgemäße Befestigungsanordnung in einer beispielhaften Anwendung zur Befestigung eines Knochenriegels an einem Knochen im Längsschnitt;
- Fig. 2: einen Querschnitt durch die Befestigungsanordnung in Fig. 1 entlang der Linie II-II in Fig. 1;
- Fig. 3: einen Querschnitt durch die Befestigungsanordnung in Fig. 1 entlang der Linie III-III in Fig. 1;
- Fig. 4: einen Ausschnitt aus der Befestigungsanordnung in Fig. 1 in einer der Fig. 1 entsprechenden Darstellung, die die Funktion der Befestigungsanordnung beim Eindrehen der Schraube in den Untergrund in einem ersten Stadium zeigt;
- Fig. 5: eine der Fig. 4 entsprechende Darstellung, in der die Schraube so weit in den Untergrund eingedreht ist, daß der Kopf der Schraube gerade mit der Hülse der Befestigungsanordnung in Eingriff kommt; und
- Fig. 6: eine schematische Darstellung der Beweglichkeit der Hülse um die Symmetrieachse der Bohrung des Befestigungsteils.

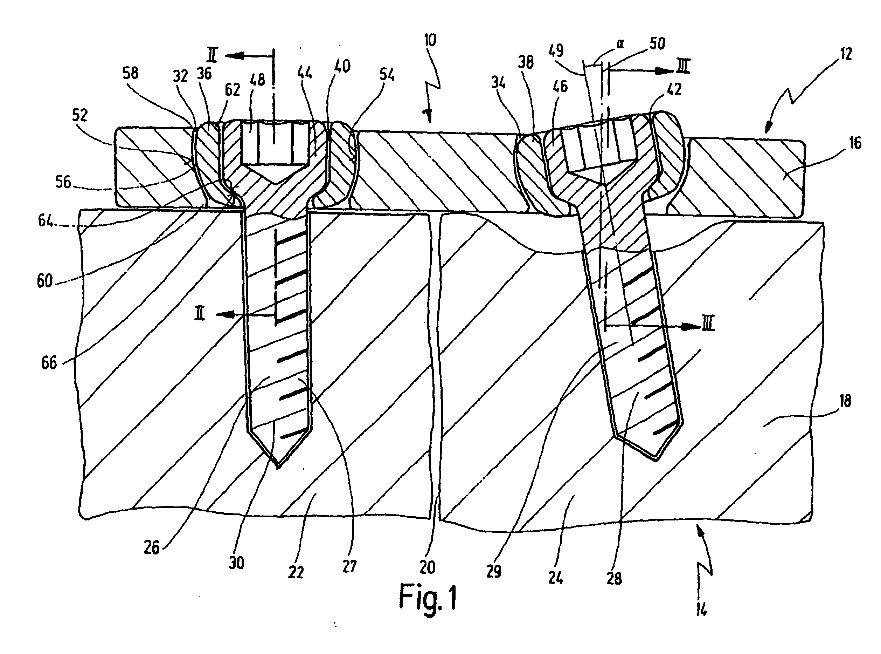

In Figuren 1 bis 3 ist eine mit dem allgemeinen Bezugszeichen 10 versehene Befestigungsanordnung zum Befestigen eines Befestigungsteils 12 an einem Untergrund 14 dargestellt.

In dem in den Figuren dargestellten Ausführungsbeispiel ist das Befestigungsteil 12 ein Riegel 16 in Form einer etwa rechteckigen länglichen Platte, der an einem Knochen 18 befestigt wird, der eine Fraktur 20 aufweist. Mittels des Riegels 16 werden zwei durch die Fraktur 20 getrennte Knochenteile 22 und 24 zur Heilung der Fraktur 20 aneinander fixiert.

Der Riegel 16 ist mittels einer ersten Schraube 26 sowie einer zweiten Schraube 28 an dem Knochenteil 22 bzw. dem Knochenteil 24 befestigt. Die erste Schraube 26 und die zweite Schraube 28 sind Knochenschrauben, die jeweils ein an einem Schaft 27 bzw. 29 der Schraube 26 bzw. 28 ausgebildetes Gewinde 30 aufweisen, das beim Eindrehen der Schrauben 26 bzw. 28 ein entsprechendes Gewinde in die Knochenteile 22 bzw. 24 schneidet.

In dem Riegel 16 ist eine erste Bohrung 32 und eine zweite Bohrung 34 vorgesehen, wobei in der ersten Bohrung 32 eine erste Hülse 36 und in der zweiten Bohrung 34 eine zweite Hülse 38 aufgenommen ist.

Die erste Hülse 36 weist eine Aufnahme 40, und die zweite Hülse 38 weist eine Aufnahme 42 auf, die der Aufnahme eines Kopfes 44 der Schraube 26 bzw. der Aufnahme eines Kopfes 46 der Schraube 28 dienen. Der Kopf 44 der Schraube 26 und der Kopf 46 der Schraube 28 weisen jeweils einen Innensechskant 48 auf, der zur formschlüssigen Aufnahme eines Inbus-Schlüssels zum Eindrehen bzw. Ausdrehen der Schrauben 26 bzw. 28 dient.

Die Hülsen 36 bzw. 38 sind in der Bohrung 32 bzw. 34 in mehreren, in dem gezeigten Ausführungsbeispiel in allen Raumrichtungen verkippbar gelagert. Diese Verkippbarkeit der Hülsen 36 und 38 besteht jedoch nur dann, wenn die Köpfe 44 bzw. 46 noch nicht in der Aufnahme 40 bzw. 42 der Hülse 36 bzw. 38 aufgenommen sind. In dem in Figuren 1 bis 3 dargestellten Zustand, in dem die Köpfe 44 bzw. 46 in den Aufnahmen 40 bzw. 42 versenkt und die Schraube 26 bzw. 28 in dem Knochen 18 eingeschraubt sind, sind die Hülsen 36 bzw. 38 fixiert. Im in Figuren 1 bis 3 dargestellten endbefestigten Zustand ist das Befestigungsteil 12 vollkommen unbeweglich mit dem Untergrund 14 verbunden.

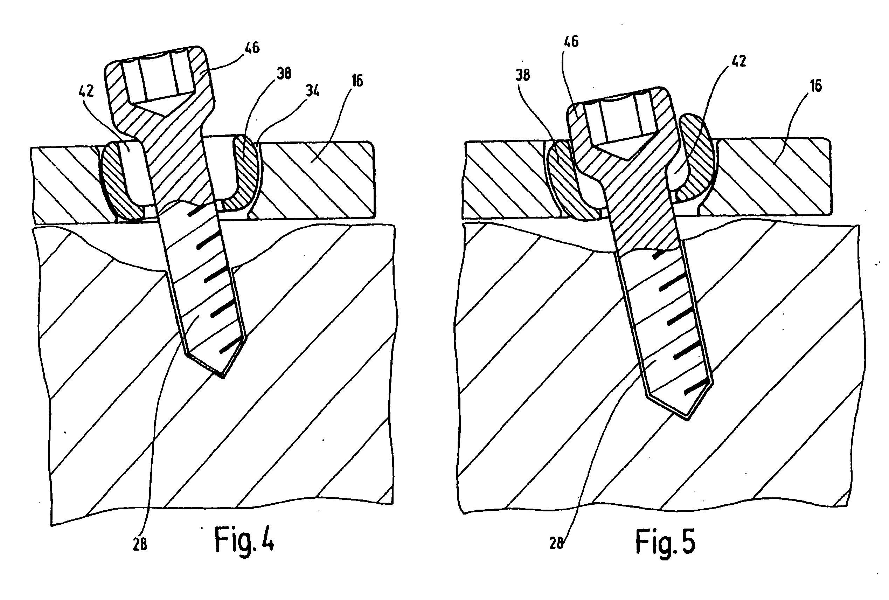

Die freie Verkippbarkeit der Hülse 36 bzw. 38 besteht jedoch so lange, wie die Köpfe 44 bzw. 46 der Schrauben 26 bzw. 28 noch nicht in die Aufnahme 40 der Hülse 36 bzw. in die Aufnahme 42 der Hülse 38 eingreifen, wie am Beispiel der Schraube 28 und der Hülse 38 in Fig. 4 dargestellt ist.

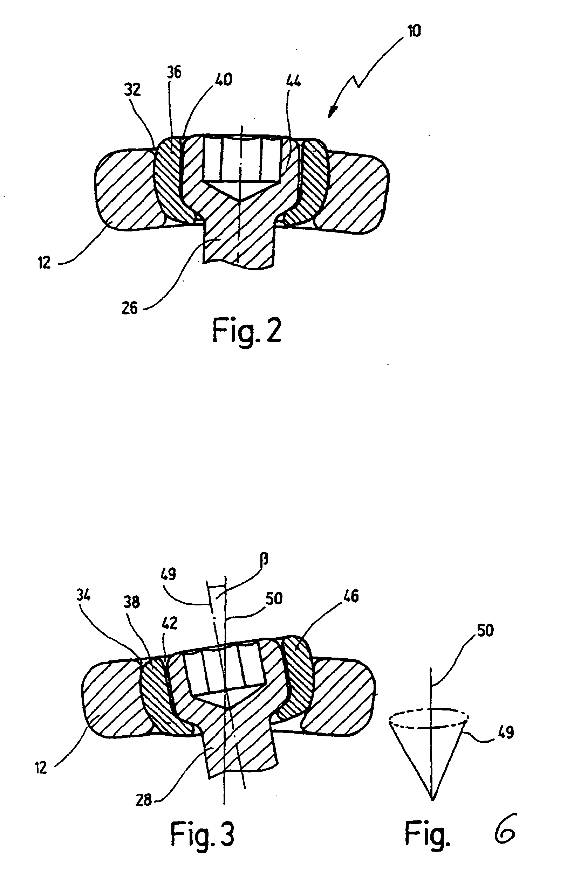

Bei dem Ausführungsbeispiel gemäß Figuren 1 bis 3 ist die Hülse 38 in der Bohrung 34 des Riegels 16 derart verkippt, daß eine Symmetrieachse 49 der Hülse 38 gegen eine Symmetrieachse 50 der Bohrung 34 bezüglich der Längsachse des Riegels 16 um einen Winkel α verkippt ist (vgl. Fig. 1) und in einer Richtung quer zur Längsachse des Riegels 16 um einen Winkel β (vgl. Fig. 3). Die freie Verkippbarkeit der Hülse 38 bewirkt, daß, wenn die Schraube 28, wie in Figuren 1, 4 und 5 dargestellt ist, schräg in den Knochen 18 eingeschraubt wird, sich die Hülse 38 selbsttätig in der Bohrung 34 so positioniert, daß die Symmetrieachse 49 der Hülse 38 mit der Symmetrieachse der Schraube 28 zusammenfällt, so daß ein Verklemmen der Schraube 28 beim Eindrehen in den Knochen 18 mit der Hülse 38 vermieden wird.

Im vollkommen eingedrehten Zustand ist der Kopf 46 der Schraube 28 formschlüssig in der Aufnahme 42 der Hülse 38 aufgenommen. Das formschlüssige Eingreifen des Kopfes 46 in der Aufnahme 42 der Hülse 38 wird stets beim Eindrehen der Schrauben 26 und 28 automatisch erreicht, unabhängig davon, ob die Schraube 28 schräg in den Knochen 18 eingeschraubt wird, oder, wie im Falle der Schraube 26 dargestellt ist, ob die Schraube 26 lotrecht in den Knochen 18 eingeschraubt wird.

Im vollkommen eingeschraubten Zustand der Schraube 26 bzw. 28 sitzt der Kopf 44 der Schraube 26 bzw. der Kopf 46 der Schraube 28 auf Preßsitz in der Aufnahme 40 bzw. 42 der Hülse 36 bzw. 38, wodurch die Hülse 36 und die Hülse 38 gegen die Wand der Bohrung 32 bzw. 34 gepreßt werden.

Die Hülse 36 bzw. die Hülse 38 sind in der Bohrung 32 bzw. 34 über einen vollen Azimutalwinkel von 360° in einem Winkelbereich von 0° bis zumindest 45° verkippbar (vgl. Fig. 6). In Fig. 6 ist dies für einen Kippwinkel von etwa 10° gezeigt.

Bei dem in Figuren 1 bis 3 gezeigten Ausführungsbeispiel sind die Hülsen 36 und 38 unmittelbar in den Bohrungen 32 bzw. 34 gelagert, und zwar sind die Hülsen 36 bzw. 38 in den Bohrungen 32 bzw. 34 formschlüssig aufgenommen. Aufgrund des Formschlusses sind die Hülsen 36 und 38 in den Bohrungen 32 und 34 unverlierbar gehalten. Eine Innenfläche 52 der Bohrung 32 des Riegels 16 und eine Außenfläche 54 der Hülse 36 sind dazu sphärisch gewölbt ausgebildet, wobei die Innenfläche 52 der Bohrung 32 sphärisch konkav und die Außenfläche 54 der Hülse 36 sphärisch konvex ausgebildet ist. Dabei befindet sich ein Bereich 56 größten Durchmessers der Innenfläche 52 und der Außenfläche 54 zwischen einem oberen Rand 58 der Bohrung 32 und einem unteren Rand 60 der Bohrung 32. Durch diese Ausgestaltung ist die Hülse 36 unverlierbar, jedoch in allen Raumrichtungen frei verkippbar in der Bohrung 32 aufgenommen. Die Hülse 38 und die Bohrung 34 sind entsprechend ausgestaltet.

Ferner ist ein oberer Rand 62 der Hülse 36 abgerundet ausgebildet, ebenso bei der Hülse 38. An dem Kopf 44 der Schraube 26 ist (wie an dem Kopf 46 der Schraube 28) in einem sich radial nach unten verjüngenden Umfangsbereich eine Abstützfläche 64 ausgebildet, die sich in dem in Fig. 1 dargestellten befestigten Zustand des Riegels 16 auf einer etwa komplementär dazu ausgebildeten Abstützfläche 66 der Hülse 36 abstützt. Insgesamt liegt der Kopf 44 in in der Aufnahme 40 der Hülse 36 vollkommen versenkter Lage flächig in der Aufnahme 40 der Hülse 36 an. Die gegeneinanderdrückenden, schräg ausgebildeten Abstützflächen 64 bzw. 66 bewirken dabei eine leichte Dehnung der Hülse 36, durch die die Verankerung und Verpressung des Schraubenkopfes 44 in der Hülse 36 verbessert wird.

Wie aus Figuren 1 bis 3 hervorgeht, weist der Kopf 44 der Schraube 26 außerdem etwa die gleiche Höhe auf wie die Aufnahme 40 der Hülse 36, und ebenso weist die Hülse 36 in etwa die gleiche Höhe auf wie die Bohrung 32, so daß der obere Rand 62 der Hülse 36 mit der Oberseite des Kopfes 44 der Schraube 26 und der Oberseite des Riegels 16 eine im wesentlichen gleichmäßige Fläche bildet.

Bei dem mit Bezug auf Figuren 1 bis 3 beschriebenen Ausführungsbeispiel sind die Hülsen 36, 38 einstückig aus Metall oder aus Kunststoff gefertigt, oder weisen einen Metallkorpus auf, wobei die jeweilige in den Hülsen 36, 38 ausgebildeten Aufnahmen 40, 42 zumindest teilweise mit Kunststoff ausgekleidet sind.

Während die Befestigungsanordnung 10 für einen speziellen Anwendungsfall im medizinischen Bereich zur Befestigung eines Riegels 16 an einem Knochen 18 beschrieben wurde, versteht es sich von selbst, daß eine derartige Befestigungsanordnung auch für technische Zwecke verwendbar ist, beispielsweise um in häuslichen Anwendungen Befestigungsteile, wie Platten, Riegel, Winkel, Halter usw., an einer Wand oder einer Decke zu befestigen.

## Patentansprüche

1. Befestigungsanordnung zum Befestigen eines Befestigungsteils (12) beispielsweise einer Platte, eines Riegels (16), eines Winkels, eines Profils, eines Beschlags, eines. Halters oder dergleichen, an einem Untergrund (14) mittels zumindest einer Schraube (26, 28), die durch zumindest eine Bohrung (32, 34) in dem Befestigungsteil (12) durchführbar und in den Untergrund (14) einschraubbar ist, wobei unmittelbar in der Bohrung (32, 34) eine in mehreren Raumrichtungen verkippbar gelagerte Hülse (36, 38), angeordnet ist, durch die die Schraube (26, 28) zum Eindrehen in den Untergrund (14) durchgeführt wird, und wobei die Hülse (36, 38) eine Aufnahme (40, 42) zum zumindest teilweisen flächigen Aufnehmen eines Kopfes (44, 46) der Schraube (26, 28) aufweist, wobei eine Innenfläche (52) der Bohrung (32, 34) und eine AuBenfläche (54) der Hülse (36, 38) etwa sphärisch gewölbt ausgebildet sind, wobei sich ein Bereich (56) größten Durchmessers der Innenfläche (52) und der Außenfläche (54) zwischen einem oberen Rand (58) der Bohrung (32, 34) und einem unteren Rand (60) der Bohrung (32, 34) befindet, und wobei die Hülse (36, 38) in die Bohrung (32, 34) formschlüssig unverlierbar eingepreßt ist, **dadurch gekennzeichnet, daß** ein Umfangsbereich des Kopfes (44, 46) der Schraube (26, 28) als Abstützfläche (64) ausgebildet ist, die sich im angezogenen Zustand der Schraube (26, 28) auf einer etwa komplementär zur Abstützfläche (64) des Kopfes (44, 46) ausgebildeten Abstützfläche (66) der Hülse (36, 38) abstützt.

2. Befestigungsanordnung nach Anspruch 1, **dadurch gekennzeichnet, daß** eine Symmetrieachse (48) der Hülse (36, 38) gegen eine Symmetrieachse (50) der Bohrung (32, 34) über vorzugsweise einen vollen Azimutalwinkel von 360° in einem Winkelbereich von 0° bis zumindest 45° verkippbar ist.

3. Befestigungsanordnung nach Anspruch 1 oder 2, **dadurch gekennzeichnet, daß** der Kopf (44, 46) der Schraube (26, 28) und die Aufnahme (40, 42) der Hülse (36, 38) eine im wesentlichen formschlüssige Verbindung bilden.

4. Befestigungsanordnung nach einem der Ansprüche 1 bis 3, **dadurch gekennzeichnet, daß** der Kopf der Schraube und die Aufnahme der Hülse komplementär zueinander konisch ausgebildet sind.

5. Befestigungsanordnung nach einem der Ansprüche 1 bis 4, **dadurch gekennzeichnet, daß** ein oberer Rand (62) der Aufnahme (40) der Hülse (36) abgerundet ist.

6. Befestigungsanordnung nach einem der Ansprüche 1 bis 5, **dadurch gekennzeichnet, daß** die Hülse (36, 38) in etwa die gleiche Höhe aufweist wie die Bohrung (32, 34), und daß der Kopf (44, 46) der Schraube (26, 28) in etwa die gleiche Höhe aufweist wie die Aufnahme (40, 42) der Hülse (36, 38).

7. Befestigungsanordnung nach einem der Ansprüche 1 bis 6, **dadurch gekennzeichnet, daß** die Hülse (36, 38) einstückig aus Metall oder aus Kunststoff gefertigt ist, oder einen Metallkorpus aufweist, wobei die Aufnahme (40, 42) der Hülse (36, 38) dann zumindest teilweise mit Kunststoff ausgekleidet ist.

8. Verwendung einer Befestigungsanordnung nach einem der Ansprüche 1 bis 7 zum Anbringen eines Gegenstandes an einer Wand, wobei das Befestigungsteil (12) ein Wandbeschlag, ein Profil oder dergleichen ist, oder zur Montage von Möbeln oder dergleichen, wobei das Befestigungsteil ein Beschlag, ein Scharnier, ein Winkel oder dergleichen ist.

## Claims

1. A fixing arrangement for fixing a fixing element (12), for example a plate, a brace (16), an angle, a profile, an armature, a retainer or the like, to a substructure (14) by means of at least one screw (26, 28) which can be passed through at least one bore (32, 34) in the fixing element (12) and can be screwed into the substructure, wherein a bushing is directly arranged in said bore (32, 34), through which said screw can be passed for screwing said screw into said substructure (14), said bushing being able to swivel in several spatial directions and wherein said bushing (36, 38) comprises a seat (40, 42) for receiving at least a partial surface of a head (44, 46) of the screw (26, 28), wherein an inner surface (52) of said bore (32, 34) and an outer surface (54) of said bushing (36, 38) are formed to be about spherically curved, wherein a region (56) of the largest diameter of the inner surface (52) and of the outer surface (54) is situated between an upper edge (58) of said bore (32, 34) and a lower edge (60) of said bore (32, 34), and wherein the bushing (36, 38) is in a form-locking manner loss-prove pressed in said bore, **characterized in that** a circumferential region of the head (44, 46) of the screw (26, 28) is configured as a support surface (64) which in the tightened condition of the screw (26, 28) rests against a support surface (66) of the bushing (36, 38) formed to be approximately complementary to the support surface (64) of the head (44, 46).

2. A fixing arrangement according to claim 1, **characterized in that** an axis of symmetry (48) of the bushing (36, 38) is tiltable relative to an axis of symmetry (50) of the bore (32, 34) within an angular range of 0° to at least 45° preferably over a full azimuth angle of 360°.

3. A fixing arrange to claim 1 or 2, **characterized in that** the head (44, 46) of the screw (26, 28) and the receiver (40, 42) of the bushing (36, 38) form a substantially form-locking connection.

4. A fixing arrangement according to one of claims 1 to 3, **characterized in that** the head of the screw and the receiver of the bushing are conically formed so as to be complementary to one another.

5. A fixing arrangement according to one of claims 1 to 4, **characterized in that** an upper edge (62) of the receiver (40) of the bushing (36) is rounded.

6. A fixing arrangement according to one of claims 1 to 5, **characterized in that** the bushing (36, 38) has substantially the same height as the bore (32, 34) and **in that** the head (44, 46) of the screw (26, 28) has substantially the same height as the receiver (40, 42) of the bushing (36, 38).

7. A fixing arrangement according to one of claims 1 to 6, **characterized in that** the bushing (36, 38) is formed in one piece from metal or plastic or has a metal body, in which the receiver (40, 24) of the bushing (36, 38) is then at least partly lined with plastic.

8. Use of a fixing arrangement according to one of claims 1 to 7 for mounting an object on a wall, wherein the fixing part (12) is a wall fitting, a profile or the like, or for assembling furniture, etc., wherein the fixing part is an armature, a hinge, an angle or the like.

## Revendications

1. Dispositif de fixation destiné à fixer une pièce de fixation (12) telle qu'une plaque, une barre (16), une équerre, un profilé, une ferrure, un support ou analogue, sur un substrat (14) au moyen d'au moins une vis (26, 28) qui peut être passée dans au moins un alésage (32, 34) de la pièce de fixation (12) et vissée dans le substrat (14), une douille (36, 38) apte à pivoter dans plusieurs directions dans l'espace étant directement disposée dans l'alésage (32, 34), au moyen de laquelle la vis (26, 28) est passée pour être vissée dans le substrat (14), et la douille (36, 38) étant pourvue d'un logement (40, 42) destiné à recevoir au moins partiellement la surface d'une tête (44, 46) de la vis (26, 28), une surface intérieure (52) de l'alésage (32, 34) et une surface extérieure (54) de la douille (36, 38) étant prévues avec un bombage sensiblement sphérique, une zone (56) de plus grand diamètre de la surface intérieure (52) et de la surface extérieure (54) étant située entre un bord supérieur (58) de l'alésage (32, 34) et un bord inférieur (60) de l'alésage (32, 34), et la douille (36, 38) étant emmanchée de manière imperdable par correspondance de forme dans l'alésage (32, 34), **caractérisé en ce qu'**une zone périphérique de la tête (44, 46) de la vis (26, 28) est réalisée comme une surface d'appui (64) qui, en état de serrage de la vis (26, 28), repose sur une surface d'appui (66) de la douille (36, 38) sensiblement complémentaire à la surface d'appui (64) de la tête (44, 46).

2. Dispositif de fixation selon la revendication 1, **caractérisé en ce qu'**un axe de symétrie (48) de la douille (36, 38) peut être pivoté par rapport à un axe de symétrie (50) de l'alésage (32, 34) dans une plage angulaire comprise entre 0° et au moins 45°, préférentiellement sur un angle azimutal entier de 360°.

3. Dispositif de fixation selon la revendication 1 ou la revendication 2, **caractérisé en ce que** la tête (44, 46) de la vis (26, 28) et le logement (40, 42) de la douille (36, 38) forment une liaison mécanique sensiblement solidaire.

4. Dispositif de fixation selon l'une des revendications 1 à 3, **caractérisé en ce que** la tête de la vis et le logement de la douille sont de formes coniques complémentaires l'une à l'autre.

5. Dispositif de fixation selon l'une des revendications 1 à 4, **caractérisé en ce qu'**un bord supérieur (62) du logement (40) de la douille (36) est arrondi.

6. Dispositif de fixation selon l'une des revendications 1 à 5, **caractérisé en ce que** la douille (36, 38) présente sensiblement la même hauteur que l'alésage (32, 34), et **en ce que** la tête (44, 46) de la vis (26, 28) présente sensiblement la même hauteur que le logement (40, 42) de la douille (36, 38).

7. Dispositif de fixation selon l'une des revendications 1 à 6, **caractérisé en ce que** la douille (36, 38) est fabriquée d'une seule pièce en métal ou en matière plastique, ou comporte un corps métallique, le logement (40, 42) de la douille (36, 38) étant alors au moins partiellement revêtu de matière plastique.

8. Utilisation d'un dispositif de fixation selon l'une des revendications 1 à 7 pour l'application d'un objet contre un mur, la pièce de fixation (12) étant une ferrure murale, un profilé ou analogue, ou pour le montage de meubles ou analogues, la pièce de fixation étant une ferrure, une charnière, une équerre ou analogue.
